# EUROPEAN PATENT SPECIFICATION

(11) **EP 3 664 685 B1**
(45) Date of publication and mention of the grant of the patent: **19.04.2023**
(21) Application number: 18844827.8
(22) Date of filing: 25.07.2018
(51) Int. Cl.: A61B 1/00, A61B 1/313, A61B 1/05, A61B 1/06

(54) **A TWO-PIECE RIGID MEDICAL SURGERY ILLUMINATING DEVICE**
ZWEITEILIGE STARRE BELEUCHTUNGSVORRICHTUNG FÜR DIE MEDIZINISCHE CHIRURGIE
DISPOSITIF D'ÉCLAIRAGE DE CHIRURGIE MÉDICALE RIGIDE À DEUX PIÉCES

(30) Priority: 08.08.2017 US 201762542327 P
(43) Date of publication of application: 17.06.2020
(73) Proprietor: 270 Surgical Ltd., 4250212 Netanya (IL)
(72) Inventor: LEVY, Avraham, 4691000 Kfar Shmaryahu (IL); SALMAN, Golan, 3030000 Atlit (IL); AIZENFELD, Amram, 1924500 Ramot Menashe (IL); LEVI, Moshe, 5590000 Ganey Tikva (IL)
(74) Representative: Schulz Junghans Patentanwälte PartGmbB
(86) International application number: PCT/IL2018/050826
(87) International publication number: WO 2019/030747

(56) References cited:
- WO-A1-2014/186521
- WO-A1-2016/084521
- WO-A1-2016/084521
- WO-A1-2016/181404
- WO-A1-2018/060985
- US-A1- 2014 142 381
- US-A1- 2014 142 381
- US-A1- 2014 364 691

## Description

### FIELD OF THE INVENTION

The present invention generally relates to the field of medical instruments designed to capture images from inside the patient's body.

### BACKGROUND OF THE INVENTION

A laparoscope is a device utilized to perform operations in the abdomen or pelvis through small incisions with the aid of a camera. It can either be used to inspect and diagnose a condition or to perform surgery. In some cases, procedures which involve inspection of a region inside confined area or a specific body cavity or organ, may also involve an endoscope.

There are multiple different types of endoscopes and laparoscope, depending on the area in which the device is used and the procedure's type. In some cases, a laparoscope may comprise one camera utilized to capture the field of view of the endoscope.

A laparoscope is likely to be assembled in an elongated tubular member in which the camera is located, and elongated handle comprising the electrical circuitry. In most cases, Gastrointestinal endoscope is inserted into the patient's body via the body's natural orifices, while laparoscopes are inserted via a trocar. The elongated member may be elastic, which enables maneuvering it in the patient's body. The elongated member may be rigid, for protecting the circuitry and sensors. When all the cameras are in the rigid elongated member, assembling the optical components and the electrical circuitry may be complex due to physical constraints of the member, and some lenses or other optical or electrical components may be damaged.

US 2014/364691 A1 discloses an electronic circuit board assembly for a tip section of a multiple viewing elements endoscope, the assembly including a base board configured to carry a first metal frame to support a front looking viewing element and a second metal frame to support a side looking viewing element; and a flexible illumination circuit board comprising a front foldable panel configured to carry three sets of front illuminators for essentially illuminating the field of view (FOV) of the front looking viewing element, and a side foldable panel configured to carry a set of side illuminators for essentially illuminating the field of view (FOV) of the side looking viewing element.

US 2017/258302 discloses an endoscope includes: a first image acquisition portion configured to acquire a first object image from a first direction; a second image acquisition portion configured to acquire a second object image from a second direction different from the first direction; first illumination light emission portions provided on a straight line with the first image acquisition portion, the first image acquisition portion being interposed between the first illumination light emission portions, and arranged along a line orthogonal to an optical axis of the first image acquisition portion; and second illumination light emission portions lined up and arranged on a plane where the second image acquisition portion is arranged at the insertion portion, and a lining direction of the first illumination light emission portions and a lining direction of the second illumination light emission portions are arranged at positions to be a twisted relation.

### SUMMARY OF THE INVENTION

The present invention discloses a thin medical imaging device comprising a rigid elongated member and a rigid distal member provided in a width of approximately 5.0 to 15 millimeters, and which can be connected to the rigid section in a rigid manner. The distal member comprises a front camera located on a front planar surface of the distal member and a first side camera located on a first lateral surface of the distal member. The medical imaging device also comprises a second side camera located on a second lateral surface of the distal member.

According to the invention, the first side camera is closer to the front planar surface than the second side camera is.

In some cases, the working distance of the front camera, the second side camera and the first side camera may be in a range of 5-150 millimeters. In some cases, the second side camera and the first side camera have a field of view of between 80-120 degrees. In some aspects of the disclosed subject matter the front camera may be located in the center of the front planar surface. In other aspects of the disclosed subject matter the front camera front surface may create an angle smaller than 90 degrees from a longitudinal axis of the rigid elongated member.

In some cases, the distal member may further comprise apertures shaped to house and secure the first side camera, the front camera and the second side camera. In some cases, said apertures may comprise opaque walls located near the front camera, the first side camera, and the second side camera. The distal member may further comprise a front illumination module for illuminating the area captured by the front camera a illumination module for illuminating the area captured by the second side camera, and an illumination module for illuminating the area captured by the first side camera. Thus, the front illumination module may comprise at least two light sources proximal to the front camera, wherein each of the illumination source may differ than the other in illumination characters and in size cases. In some cases, the front camera may not be positioned in the center of the front surface.

In possible embodiments of the disclosed subject matter, the second side camera and/or the first side camera may be tilted relative to the longitudinal axis of the rigid member. In some cases, the front camera may also be tilted relative to the longitudinal axis of the rigid member. In some other cases, the second side camera and the front camera may be tilted relative to the longitudinal axis of the rigid member. In possible embodiments of the disclosed subject matter the second side camera and the first side camera may be located at the upper half of the distal tip. In some cases, the field of view of the second side camera may continue in a sequence manner from the field of view of the front camera. In some cases, the field of view of the first side camera may continue in a sequence manner from the field of view of the front camera.

In some cases, some areas at the field of view of the second side camera and some areas at the field of view of front camera are overlapped. In some other cases, some areas at the field of view of the first side camera and some areas at the field of view of front camera are overlapped.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawing:
Fig. 1 demonstrates a medical imaging device comprises at least one camera, according to exemplary embodiments of the present invention;
Fig. 2 demonstrates an upper cross section of a medical imaging device comprises three cameras, according to exemplary embodiments of the disclosed subject matter;
Fig. 3 demonstrates an upper cross section of a medical imaging device comprises one front camera and two side cameras, according to figure 2;
Fig. 4 demonstrates a schematic description of a medical imaging device comprises one front camera and two side cameras, according to exemplary embodiments of the disclosed subject matter;
Fig. 5 demonstrates the camera layout, in a medical imaging comprising three cameras, according to exemplary embodiments of the disclosed subject matter;
Fig. 6A demonstrates the field of view (FOV) of three cameras in a medical imaging device and having a symmetry of overlap between the FOVs, according to exemplary embodiments of the disclosed subject matter;
Fig. 6B demonstrates three fields of view of three cameras in a medical imaging device having a different overlap symmetry between the three FOVs, according to exemplary embodiments of the disclosed subject matter;
Fig. 7A-7D shows some optional outlines of cameras in a medical imaging device comprises one front camera situated horizontally and two side cameras, according to exemplary embodiments of the disclosed subject matter;
Fig. 8A-8D shows diverse possible camera positions in a medical imaging device comprises one front camera tilted relatively to the horizontally axis and two side cameras, according to exemplary embodiments of the disclosed subject matter;
Fig. 9 demonstrates a medical imaging device comprising a rigid shaft which comprises the optical gear required to the operation of the medical imaging device, according to exemplary embodiments of the disclosed subject matter;
Fig. 10 shows cross-section a medical imaging device with two side cameras situated above the axis which crosses the distal tip exactly in the middle, according to exemplary embodiments of the disclosed subject matter;
Fig. 11A demonstrates a medical imaging device comprising a front camera situated perpendicular to a first side camera and a second side camera, according to exemplary embodiments of the disclosed subject matter, and;
Fig. 11B demonstrates a medical imaging device comprising a front camera situated perpendicular to a first side camera and a second side camera, according to exemplary embodiments of the disclosed subject matter.

### DETAILED DESCRIPTION OF THE INVENTION

The present invention discloses a thin medical imaging device which can comprise three cameras designed to aid medical procedures such as inspection and surgery procedures in the abdomen or pelvis through small incisions. In some cases, such a medical imaging device can be utilized in laparoscopy wherein the medical imaging device can be put through an incision in the body in order to perform medical procedures at the internal organs. The medical imaging device disclosed herein can comprise two section members directly connected. Said two section members may be an elongated rigid shaft tube and a distal tip. The distal tip can comprise the optical gear required for the medical procedures, and is mounted directly on the rigid shaft. In some cases, the optical gear located in the distal tip can comprise cameras, lenses and light sources required for the camera functioning.

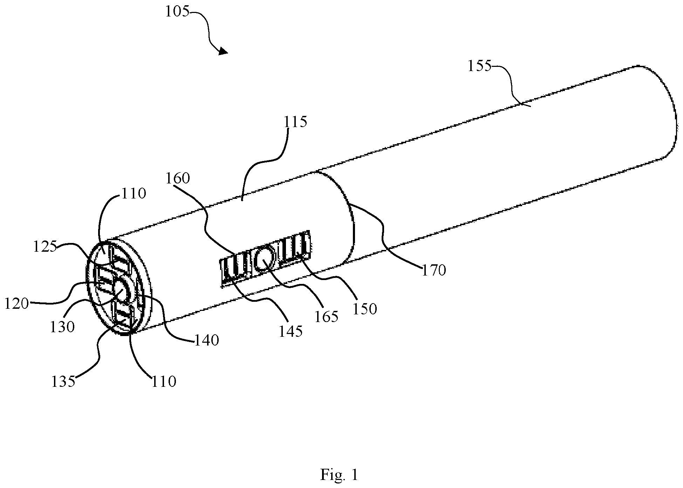

Fig. 1 demonstrates a medical imaging device comprising at least one camera, according to exemplary embodiments of the disclosed subject matter. Fig. 1 shows a medical imaging device 105 comprising a rigid shaft 155 designed to be directly connected to a distal tip 115. In such cases, the seamline 170 outlines the connection line between the rigid shaft 155 and the distal tip 115. In some cases, the rigid shaft 155 and the distal tip 115 may be connected by an adhesive material that seals the connection at the seamline 170. In some other cases, the rigid shaft 155 and the distal tip 115 may be connected by soldering. In possible embodiments of the disclosed subject matter, the rigid shaft 155 and the distal tip 115 may be connected by a screwing mechanism which fastens the rigid shaft 155 and the distal tip 115 together.

The distal tip 115 functions as a multi-camera section member designed to house at least three cameras. One of the cameras of the distal tip 115 is located in the front at the planar surface 110. Additional cameras are located at the lateral round surface of the distal tip 115. The distal tip 115 may also comprise an aperture 160 shaped to house the second side camera 165 and provide the opening required for the field of view of the second side camera 165. In some cases, the aperture 160 may be covered by a transparent layer, such as glass or plastic, to isolate the side camera 165 from the patient's tissue. In some other cases, aperture 160 may be covered by an optical window or more than one optical window.

The distal tip 115 comprises a first side camera (not shown) located at the opposite side of the distal tip 115. The aperture 160 also enables emission of light from side illuminators 150, and 145 which provide the light source of the side camera 165. In some cases, the light may be emitted by dedicated section illuminators such as light-emitting diodes, also known as LED.

The distal tip 115 may also comprise a front camera 130 situated at the center of a planar surface 110 which can house the front camera 130 and provide the opening required for the field of view of the front camera 130. The planar surface 110 also comprise front illuminators 120, 125, 135, and 140 which provide the required source of light for front camera 130. In another embodiment, the number and location of front illuminators may vary for example, less than 4 illumination modules or more wherein each illumination module has 1, 2, 3, 4 or more LED and emit different light spectrum as will explain below.

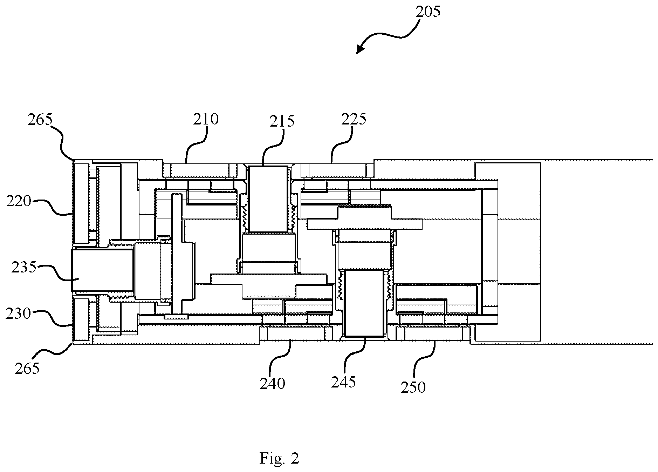

Fig. 2 demonstrates an upper cross section of a medical imaging device that comprises three cameras, according to exemplary embodiments of the disclosed subject matter. Fig. 2 shows a medical imaging device 205 comprising three cameras - front camera 235, first side camera 215 and second side camera 245. The medical imaging device 205 also comprises a tip section 265 housing the front camera 235 wherein the front camera 235 comprises a lens assembly for capturing a predefined front field of view. In some exemplary cases, the field of view of the front camera 235 is of at least 100 degrees with a working distance of approximately 15 to 150 millimeters. The front camera 235 can be positioned on a surface of a distal end of the distal tip 265. The medical imaging device 205 may also comprise two illumination modules 220, and 230 which can be LED emitting light required for the operation of the front camera 235. In some cases, the number of illumination modules are more than two. Wherein each illumination module may include 1, 2, 3, 4 or more LEDs and emit different light spectrum as explained below.

The medical imaging device 205 further comprises a second side camera 245 which can be positioned within the medical imaging device 205 such that the center of said second side camera 245 may be approximately 5 to 25 millimeters from the distal tip 265 of the medical imaging device 205. The field of view of the second side camera 245 may be at least 100 degrees, with a working distance of approximately 15 to 150 millimeters. The medical imaging device 205 may also comprise two illumination modules 240 and 250 which can be LED emitting light required for operation of the second side camera 245. In some cases, the number of illumination modules may be more than two. Wherein each illumination modules may include 1, 2, 3, 4 or more LEDs and emit different light spectrum as explain below.

The first side camera 215 may be positioned at the opposite side of the second side camera 245 such that the two cameras second side camera 245 and first side camera 215 may be pointing at directions essentially opposing to one another. The center of the first side camera 215 camera may be approximately 5 to 15 millimeters from the distal tip 265 of the medical imaging device 205. The field of view of the first side camera 215 may be at least 100 degrees, with a working distance of approximately 15 to 150 millimeters. The medical imaging device 205 may also comprise two illumination modules 225, and 210 which can be LED emitting light required for operation of the first side camera 215. In some cases, the number of illumination modules are more than two. Wherein each illumination module may include 1, 2, 3, 4 or more LEDs and emit different light spectrum as explain below.

The illumination modules 220, 230, 225, 240, 250 and 210 may receive electrical power via a cable placed in the rigid shaft 155. In some embodiments of the disclosed subject matter the light emitted by the LED's may be a white light. In some other cases, a portion of the light sources of the medical imaging device 205 may be at different colors at the visible light. For example, the light source of the medical imaging device 205, may comprise LED's emitting other colors such as blue, red, yellow, green, or any combination thereof. In some cases, the light emitted by the LED's may be at the spectrum of the non-visible light. For example, a light source can provide a light at the infrared spectrum, ultra-violate, x-ray, and the like.

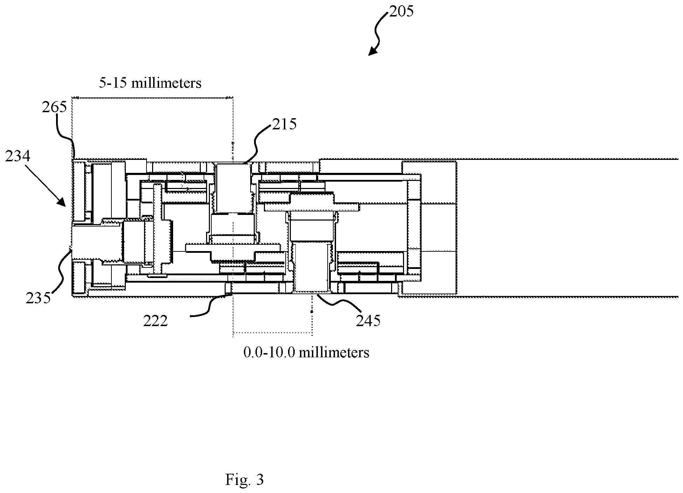

Fig. 3 demonstrates an upper cross section of a distal tip comprises one front camera and two side cameras, according to figure 2. Fig. 3 shows the distal tip 205 comprising three cameras. The front camera 235 is positioned at the front planar surface 234 located at tip edge point 330 of the distal end of the distal tip 205. In some cases, the front camera 235 may be situated closer to one of the sides of the front planar surface 234. In figure 3, the front camera 235 is located closer to the side of the second side camera 245. The medical imaging device 205 also comprises a first side camera 215 located such that the center of first side camera 215 may be located approximately 0.0 to 10.0 millimeters from tip edge point 265. Second side camera 245 located such that the center of second side camera 245 may be located approximately 0.0 to 10.0 millimeters from the center of first side camera 215 to the imaginary line continuing from the central of the first side camera 215 to point 222.

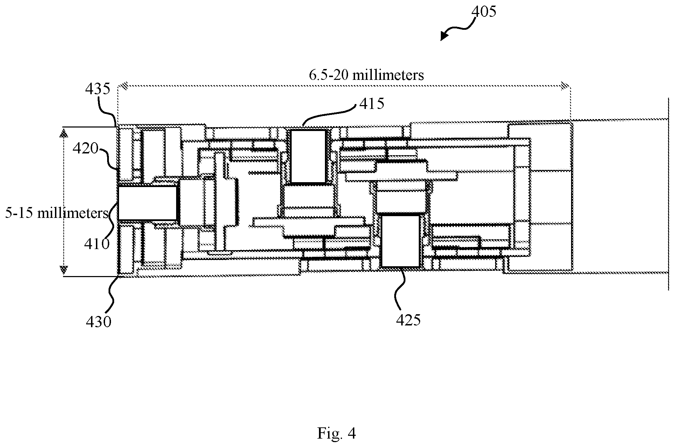

Fig. 4 demonstrates a schematic description of a distal tip comprises one front camera and two side cameras, according to exemplary embodiments of the disclosed subject matter. Fig. 4 shows a distal tip 405 comprising three cameras. A front camera 410 is positioned essentially at the center of a front planar surface 420. The distal tip 405 further comprises a second side camera 425 located at the lateral surface of the distal tip 405. The distal tip 405 further comprises a first side camera 415 located at the opposite lateral surface of the second side camera 425 such that the two cameras second side camera 425 and first side camera 415 may be pointing at directions essentially opposing to one another.

The distal tip 405 may be provided in a width of approximately 5.0 to 15 millimeters, and at the length of approximately 6.5 to 20 millimeters. In possible embodiments of the disclosed subject matter, the distance between the center of the second side camera 425 and a second edge point 430 may be substantially the same distance between the center of the first side camera 415 and a first edge point 435. Both first edge point 435 and second edge point 430 are located on the periphery of front planar surface 420. In possible embodiments of the disclosed subject matter, the distance between the center of the second side camera 425 and the edge point 430 may be shorter than the distance between the center of the first side camera 415 and the edge point 435. In possible embodiments of the disclosed subject matter, the distance between the center of the second side camera 425 and the edge point 430 may be longer than the distance between the center of the first side camera 415 and the edge point 435.

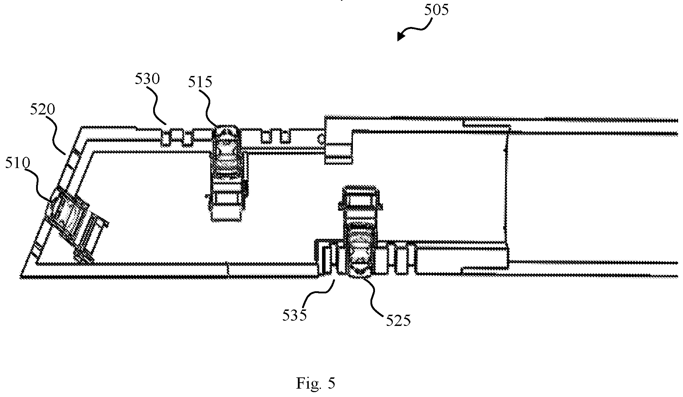

Fig. 5 demonstrates the camera layout of a distal tip in a medical imaging device comprising three cameras, according to exemplary embodiments of the disclosed subject matter. Fig. 5 shows a distal tip 505 comprising a front camera 510 situated with a bias towards the edge of the front planar surface 520. The front planar surface 520 forms an angle of less than 90 degrees from the longitudinal axis of the distal tip 505. The distal tip 505 also comprises a second side camera 525 situated at a second side aperture 535 with a bias towards one of the edges of first side aperture 535. For example, in some cases, the second side camera 525 may be situated with a bias towards left edge of the second side aperture 535. In some other cases, the second side camera 525 can be situated with a bias towards right edge of the second side aperture 535, such that the second side camera 525 may be situated away from the front planar surface 520 of the distal tip 505. In another embodiment second side camera 525 may be situated in the center of second side aperture 535.

The distal tip 505 also comprises a first side camera 515 located at of a first side aperture 530 at the opposite lateral surface of the second side camera 525. The two cameras, second side camera 525 and first side camera 515, may be pointing at directions essentially opposing to one another. In some cases, the first side camera 515 can be situated essentially at the center of first side aperture 535. In some other cases, the first side camera 515 can be situated closer to one of the edges of first side aperture 535. For example, the first side camera 515 can be situated essentially to the right edge, or essentially to the left edge of first side aperture 535.

In some cases, the walls of the distal tip 505 may be opaque and front planar surface 520, first side and second side aperture 530 and second side aperture 535, may be transparent. In another embodiment, only parts of the front planar surface 520, first side and second side aperture 530 and second side aperture 535, may be transparent.

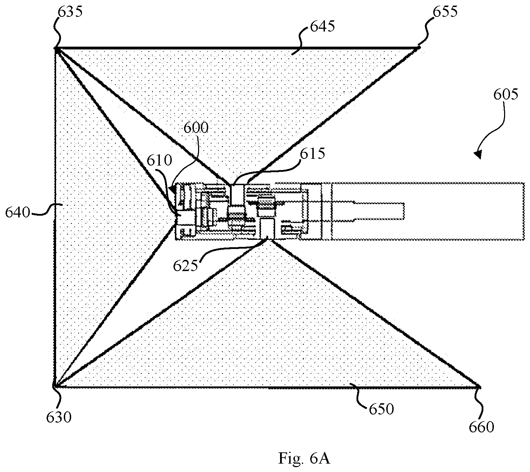

Fig. 6A demonstrates the field of view of three cameras in a medical imaging device and having a symmetry of overlap between the FOVs, according to exemplary embodiments of the disclosed subject matter. Fig. 6A shows a medical imaging device 605 comprising three cameras, a front camera 610, a second side camera 625 and a first side camera 615. The front camera 610 may be provided with a Field of View (FOV) 640. The FOV 640 can be an imaginary triangle representing the observable view of the front camera 610 and defined by the front camera 610, the point 630, and the point 635. Point 635 also outlines one of the edges of the field of view of first side camera 615, denoted as FOV 645. The FOV 645 can be the imaginary triangle representing the observable view of the first side camera 615 and defined by the first side camera 615, the point 635 and the point 655. In some cases, FOV 640 may overlap with FOV 645 at the point 635. In some other cases, FOV 645 and FOV 640 may not have any overlap. The point 630 also outlines one of the edges at the field of view of the second side camera 625, denoted as FOV 650. The FOV 650 can be the imaginary triangle representing the observable view of the second side camera 625 and created with the second side camera 625, the point 630 and the point 660. In some cases, FOV 640 may overlap with FOV 650 at the point 630. In some cases, the front camera 610 may be located on the front planar closer to the rear side camera, which is the second side camera 625, located further than the front planar 600. Thus, in cases, the front planar 600 is perpendicular to the longitudinal axis of the distal tip, and the three FOVs, 640, 645 and 650, are not equal in value a symmetry in the overlap FOV is obtained. When such symmetry is found, point 630 and point 635 have the same distance from a front planar 600.

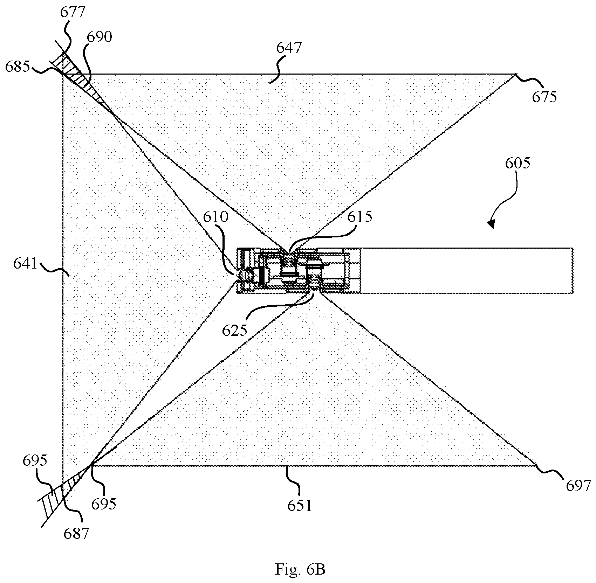

Fig. 6B demonstrates three fields of view of three cameras in a medical imaging device, according to exemplary embodiments of the disclosed subject matter. Fig. 6B shows a medical imaging device 605 comprising three cameras, the front camera 610, the second side camera 625 and the first side camera 615. The front camera 610 may be provided with a Field of View (FOV) 641. The FOV 641 can the imaginary triangle representing the observable view of the front camera 610 and defined by the front camera 610, point 677, and point 687. Point 685 outlines one of the edges of the field of view of first side camera 615, denoted as FOV 647. The FOV 647 can be the imaginary triangle representing the observable view of the first side camera 615 and defined by the first side camera 615, point 685 and point 675. In some cases, some of the seen areas of FOV 641 may be also seen in FOV 647. Thus, the first overlap FOV area 690 refers to areas seen at both FOVs, 641 and 647.

Point 697 outlines one of the edges at the field of view of the second side camera 625, denoted as FOV 651. The FOV 651 can be defined as the imaginary triangle defining the observable view of the from the second side camera 625 and created with the second side camera 625, the point 697 and the point 695. In some cases, some of the seen areas of FOV 641 may be also seen in FOV 651. Thus, the second overlap FOV area 695 refers to areas seen at both FOVs, 641 and 651.

In possible embodiments, wherein equal FOVs, 641, 647 and 651 are designed for front camera 610, first side camera 615 and second side camera 625, and when the location of each of side cameras, 615 and 625, relatively to the front camera 610 is different, first overlap FOV 690 and second overlap FOV 695 are not equal.

For example, in some cases, in a medical procedure wherein the medical imaging device 605 is utilized to inspect a certain area within a body, some of the areas of the inspected area may remain outside the FOV 651 and FOV 641. In some other cases, the FOV 641 may overlap some areas seen in FOV 651. In such cases, a person utilizing the medical imaging device 605 may be able to see one object in two different views. For example, in case a person utilizing the medical imaging device 605 sees an object located within FOV 651 and seen via the second side camera 625. In such cases, the person may be able to also see the same object, in different angle from a different point of view via first side camera 615.

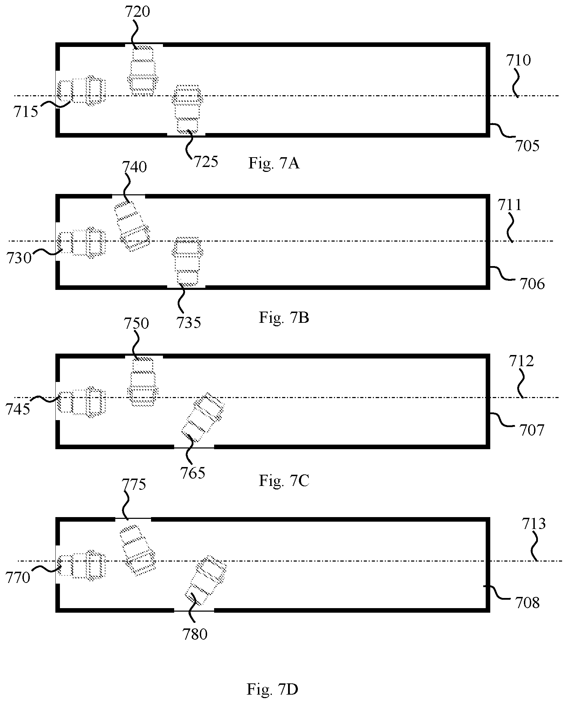

Figs. 7A-7D show some optional outlines of cameras in a medical imaging device comprises one front camera situated horizontally to a longitudinal axis of the medical imaging device and two side cameras, wherein the two side cameras locate 0.0 mm to 10.0 mm apart along the longitudinal axis of the medical imaging device, according to exemplary embodiments of the disclosed subject matter. Figs. 7A-7D show a number of schematic diagrams representing diverse embodiments of the medical imaging device. Fig. 7A shows an exemplary embodiment shown in the schematic diagram 705, in which a front camera 715 is located at the front planar panel, horizontally to a longitudinal axis 710. The schematic diagram 705 also shows side cameras 720 and 725 situated perpendicular to the longitudinal axis 711.

Fig. 7B shows an exemplary embodiment shown in the schematic diagram 706, in which a front camera 730 is located at a front planar panel, horizontally to the longitudinal axis 711 and a side camera 740 is titled relative to the longitudinal axis 711. The angle between the titled side cameras and the longitudinal axis 711 may be varied between 0.1 to 45 degrees. The schematic diagram 706 also shows a side camera 735 situated perpendicular to the longitudinal axis 711 of the medical imaging device.

Fig. 7C shows an exemplary embodiment shown in the schematic diagram 707, in which a front camera 745 is located at a front planar panel, horizontally to the longitudinal axis 712, and a side camera 765 is titled relative to the longitudinal axis 712. The angle between the titled side camera 765 and the longitudinal axis 712 may be varied between 0.1 to 45 degrees. The schematic diagram 707 also shows the side camera 750 situated perpendicular to the longitudinal axis 712 of the medical imaging device.

Fig. 7D shows an exemplary embodiment shown in the schematic diagram 708, in which a front camera 770 is located at a front planar panel, horizontally to the longitudinal axis 713, and side cameras 775 and 780 are titled relative to the longitudinal axis 713. The angle between the titled side cameras 775 and 780 and the longitudinal axis 713 may be varied between 0.1 to 45 degrees.

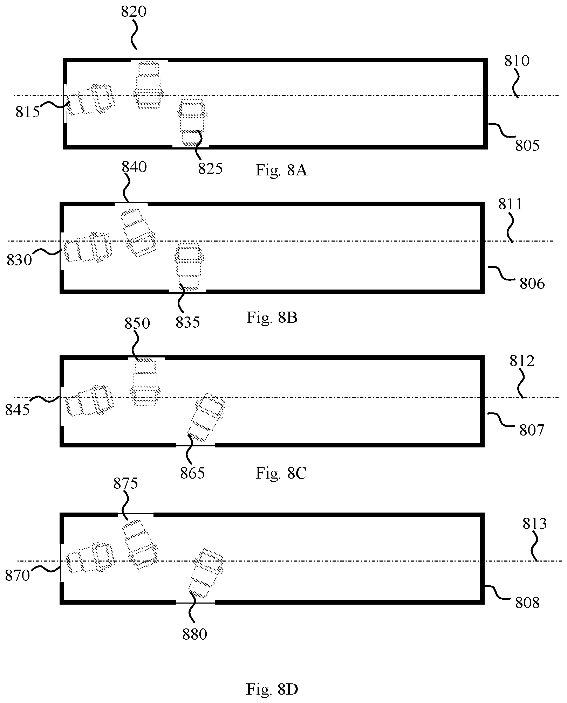

Figs. 8A-8D show diverse possible camera positions in a medical imaging device comprises one front camera tilted relatively to the horizontally axis of the device and two side cameras, wherein the two side cameras locate 0.0 mm to 10.0 mm apart along the longitudinal axis of the medical imaging device, according to exemplary embodiments of the disclosed subject matter. Figs. 8A-8D show several schematic diagrams representing diverse embodiments of the medical imaging device. Fig. 8A shows an exemplary embodiment shown in the schematic diagram 805, in which the front camera 815 is located at the front planar panel, titled relative to the longitudinal axis 810, and side cameras 820, and 825 situated perpendicular to the longitudinal axis 810. The angle between the front camera 815 and the longitudinal axis 810 may be varied between 0.1 to 45 degrees.

Fig. 8B shows an exemplary embodiment shown in the schematic diagram 806, in which a front camera 830 is located at a front planar panel, titled relative to the longitudinal axis 811, and a side camera 840 is also titled relative to the longitudinal axis 811. The angle between the side camera 840 and the front camera 830, and the longitudinal axis may be varied between 0.1 to 45 degrees. The schematic diagram 806 also shows the side camera 835 situated perpendicular to the longitudinal axis 811 of the medical imaging device.

Fig. 8C shows an exemplary embodiment shown in the schematic diagram 807, in which a front camera 845 is located at the front planar panel, titled relative to the longitudinal axis 812, and the side camera 865 situated titled relative to the longitudinal axis 812 of the medical imaging device 807. The angle between the titled side camera 865 and the front camera 845, and the longitudinal axis may be varied between 0.1 to 45 degrees. The schematic diagrams 807 also shows the side camera 850 situated perpendicular to the longitudinal axis 812 of the medical imaging device 807.

Fig. 8D shows an exemplary embodiment shown in the schematic diagram 808, in which a front camera 870 is located at the front planar panel, titled relative to the longitudinal axis 813 of the medical imaging device 808. The side cameras 875 and 880 are also titled relative to the longitudinal axis 813. The angle between the titled side cameras 875 and 880, the front camera 870 and the longitudinal axis 813 may be varied between 0.1 to 45 degrees. In some embodiments of the present invention, the titled cameras may be tilted to more than one side. For example, in one possible embodiment of the disclosed subject matter, the front camera 870 can be tilted to one side, relative to the longitudinal axis 813. In another possible embodiment of the disclosed subject matter, the front camera 870 may be tilted to the opposite side of the longitudinal axis 813

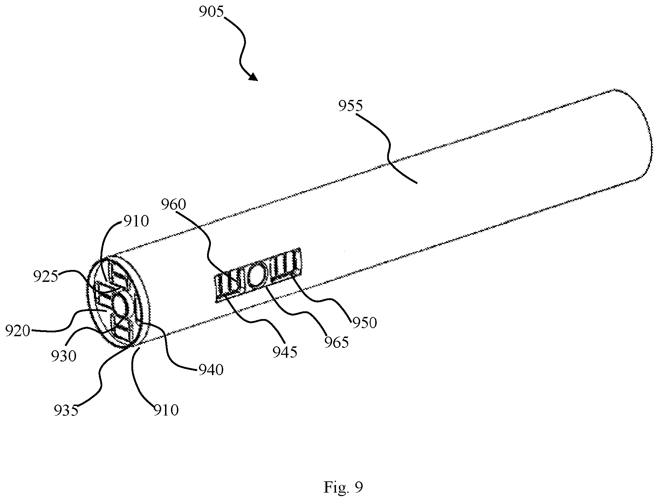

Fig. 9 demonstrates a medical imaging device comprising a rigid shaft which comprises an optical gear required for the operation of the medical imaging device, according to exemplary embodiments of the disclosed subject matter. Fig. 9 shows a medical imaging device 905 comprising one rigid shaft 955 without a distal tip. The rigid shaft 905 may function as a multi-camera section member designed to house at least one camera. In some cases, the cameras may be positioned at the edge of the rigid shaft 955 located in the front at the planar surface 910. In some other cases, the cameras may be located at the cylindrical round surface of the rigid shaft 955 at a distance of 6.5 to 40 mm from front panel 910. In yet another embodiment, a front camera may be positioned at the edge of the rigid scope at front panel 910 and one or more side cameras may be located at the cylindrical round surface of the rigid shaft 955 at a distance of 6.5 to 40 mm from front panel 910.

The rigid shaft 955 also comprises an aperture 960 shaped to house a second side camera 965 and provide the field of view operatively required for the second side camera 965. In some embodiments of the disclosed subject matter the rigid shaft 955 may comprise a first side camera (not shown) located at the opposite side of the rigid shaft 955 within a first side aperture (not shown). The aperture 960 also houses side illumination module 950, and 945 which provide the light source of the side camera 965. In some cases, the light source may be emitted by dedicated illuminators such as light-emitting diode, also known as LED. In some cases, each illumination module has 1, 2, 3, 4 or more LED and emit different light spectrum as will explained above.

The rigid shaft 955 may also comprise a front camera 930 which may be situated closer to one of at the center of a planar surface 910 which can house the front camera 930 and provide the field of view operatively required for front camera 930. The planar surface 910 also comprise front illuminators' sets 920, 925, 935, and 940 which provide the required source of light for front camera 930. In some cases, each illumination module 1, 2, 3, 4 or more LED and emit different light spectrum as will explained above. In another embodiment, front camera 930 may be situated at the center of the front planar surface 910.

In some cases, the rigid shaft 955 may be prepared as a one-piece. For example, rigid shaft 955 may be prepared by a molding process. In some cases, the preparation process of the rigid shaft 955 may also comprise a milling process for creating the apertures for the cameras, the rounded surfaces, the planar surfaces, the room for the cameras, and the like.

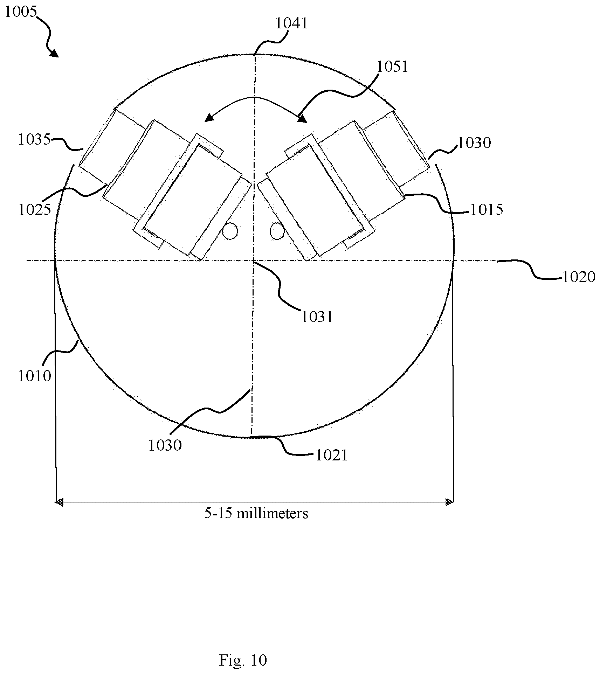

Fig. 10 shows cross-section of a medical imaging device with two side cameras situated above the axis above the axis which crosses the distal tip exactly in the middle., according to exemplary embodiments of the disclosed subject matter. Fig. 10 shows a cross-section of medical imaging device 1005 demonstrated with perpendicular axis 1030 and horizontal axis 1020. The medical imaging device 1005 comprises a distal tip 1010 provided in a width of approximately 5.0 to 15.0 millimeters, and two side cameras, a first side camera 1025 and a second side camera 1015. In some embodiments of the disclosed subject matter, the two side cameras, first side camera 1025 and a second side camera 1015 may be located above the horizontal axis 1020. The horizontal axis 1020 may cross the distal tip 1010 in the middle such that, the distance between the topmost point 1041 and center point 1031 may be equal to the distance between center point 1031 and bottom point 1021. In some cases, the first side camera 1025 and a second side camera 1015 may be tilted upwardly such that a first aperture 1035 may allow the first side camera 1025 to capture an external view of one side of the distal tip 1010. Similarly, a second aperture 1030 may allow the second side camera 1015 to capture an external view of another side of the distal tip 1010. The external views captured by the first side camera 1025 and the external view captured by the second side camera 1015 may be at directions essentially opposing to one another.

In some embodiments of the disclosed subject matter, the second side camera 1015 and the first side camera 1025 may be situated in parallel to the horizontal axis 1020. In such cases, the first aperture 1035 may also be located downwardly to allow the first side camera 1025 to capture some of the external view of one side of the distal tip 1010. In such cases, the second aperture 1030 may also be located downwardly to allow the second side camera 1015 to capture some of the external view of one side of the distal tip 1010. In some cases, the angle between the first side camera 1025 and the second side camera 1015, as shown in arrow 1051 may be 180° one to another, such that the first side camera 1025 and the second side camera 1015 may be situated in parallel to the horizontal axis 1020. In some other cases, the angle between the first side camera 1025 and the second side camera 1015, as shown in arrow 1051 may be beneath 180° one to another. In some other cases, the angle between the first side camera 1025 and the second side camera 1015, as shown in arrow 1051 may be wider than 180°.

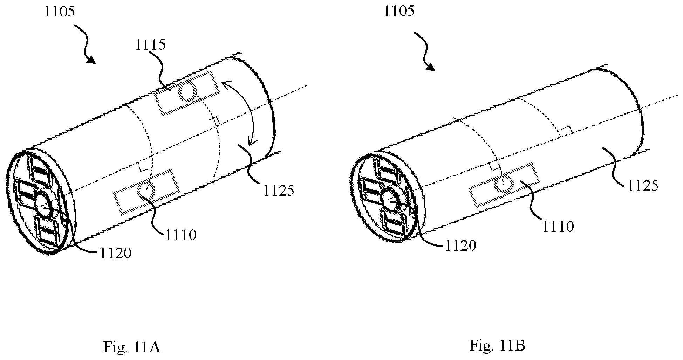

Fig. 11A demonstrates a medical imaging device comprising a front camera situated perpendicular to a first side camera and a second side camera, according to exemplary embodiments of the disclosed subject matter. Fig. 11A shows a medical imaging device 1105 comprising a front camera 1120, a first side camera 1115 and a second side camera 1110. The first side camera 1115 and the second side camera 1110 may be configured to point perpendicularly to one another, with an angle of essentially 90 degrees apart in the surface of a distal tip section 1125.

In some possible embodiments of the disclosed subject matter, the first side camera 1115 and the second side camera 1110 may be positioned in more than 90 degrees apart in the cylindrical surface of the tip section 1125, for example 120-150 degrees apart or 150-180 degrees apart. In yet another e possible embodiment of the disclosed subject matter, the first side camera 1115 and the second side camera 1110 may be positioned in less than 90 degrees apart in the cylindrical surface of the tip section 1125, such as 90-45 degrees apart or 45-25 degrees apart.

Fig. 11B demonstrates a medical imaging device comprising a front camera situated perpendicular to a first side camera and a second side camera, according to exemplary embodiments of the disclosed subject matter. Fig. 11B shows a medical imaging device 1105 comprising a front camera 1120, a first side camera 1115 and a second side camera 1110. In some cases, the front camera 1120 and the second side camera 1110 may be essentially perpendicular to one another, and have, correspondingly, perpendicular fields of view. The front camera 1120 may be essentially perpendicular to the first side camera (not shown). The first and second side cameras 1110 can be pointing perpendicularly to one another, and be positioned essentially 180 degrees apart in the cylindrical surface of the tip section 1125, in opposite sides of the cylindrical surface of the tip section 1125, such that the first side camera (not shown) and the second side camera 1110 may point in opposite directions.

The invention is defined in the appended claims.

## Claims

1. A medical imaging device (205), comprising:
a rigid elongated member (155);
a distal member (115) provided in a width of approximately 5.0 to 15 millimeters said distal member (115) comprises a front camera (130) located on a front planar surface of the distal member (115), a first side camera (215) located on a first lateral surface of the distal member (115) and a second side camera (245) located on a second lateral surface of the distal member (115, 265) and wherein the distal member (115, 265) is directly connected to the rigid elongated member (155) in a rigid manner;
**characterised in that** the first side camera (215) is located at a position closer to the front planar surface than the second side camera (245) is.

2. The medical imaging device of claim 1, wherein each of the working distance of the front camera (130), the second side camera (245) and the first side camera (215) is in a range of 5-150 millimeters.

3. The medical imaging device of claim 1, wherein each of the second side camera (245) and the first side camera (215) has a field of view of between 80-120 degrees.

4. The medical imaging device of claim 1, wherein the first side camera (215) and the second side camera (245) can be pointing perpendicularly to one another, and be positioned essentially 180 degrees or less apart in the cylindrical surface of the distal tip (1125), in opposite sides of the cylindrical surface of said distal tip (115).

5. The medical imaging device (205) of claim 3, wherein some areas at the field of view of the first side camera (215) and some areas at the field of view of front camera (130) are overlapped.

6. The medical imaging device (205) of claim 3, wherein some areas at the field of view of the second side camera (245) and some areas at the field of view of front camera (130) are overlapped.

7. The medical imaging device (205) of claim 1, wherein the distal member (115) and the rigid elongated member (155) are directly connected using a mechanism selected from a group comprising adhesive material, soldering, and screwing mechanism.

8. The medical imaging device (205) of claim 1, wherein the distal member (115) further comprises a front illumination module (220, 230) for illuminating the area captured by the front camera (130) and two first side illumination modules for illuminating the area captured by the first side camera (215).

9. The medical imaging device (205) of claim 8, wherein a front illumination module (220,230) comprises two light sources on both sides of the front camera (130), wherein one light source is bigger than the other light source in case the front camera (130) is not positioned in the center of the front surface.

10. The medical imaging device (205) of claim 1, wherein the second side camera (245) is tilted relative to the longitudinal axis of the rigid member (155).

11. The medical imaging device (205) of claim 1, wherein the front camera (130) is tilted relative to the longitudinal axis of the rigid member (155).

12. The medical imaging device (205) of claim 1, wherein the second side camera (245) and the front camera (130) are tilted relative to the longitudinal axis of the rigid member (155).

## Patentansprüche

1. Ein medizinisches bildgebendes Gerät (205), umfassend:
ein starres langgestrecktes Bauteil (155);
ein ungefähr 5.0 bis 15 Millimeter breites distales Bauteil (115), mit , wobei besagtes distale Bauteil (115) eine vordere Kamera (130), die sich auf einer vorderen ebenen Oberfläche des distalen Bauteils (115) befindet, eine erste seitliche Kamera (215), die sich auf einer ersten seitlichen Oberfläche des distalen Bauteils (115) befindet, und eine zweite seitliche Kamera (245), die sich auf einer zweiten seitlichen Oberfläche des distalen Bauteils (115, 265) befindet, umfasst, und wobei das distale Bauteil (115, 265) direkt mit dem starren langgestreckten Bauteil (155) starr verbunden ist;
**dadurch gekennzeichnet, dass** die erste seitliche Kamera (215) an einer Position angeordnet ist, die näher an der vorderen ebenen Oberfläche als die zweite seitliche Kamera (245) liegt.

2. Das medizinische bildgebende Gerät nach Anspruch 1, wobei der Arbeitsabstand der vorderen Kamera (130), der zweiten seitlichen Kamera (245) und der ersten seitlichen Kamera (215) jeweils in einem Bereich von 5-150 Millimetern liegt.

3. Das medizinische bildgebende Gerät nach Anspruch 1, wobei jede der zweiten seitlichen Kamera (245) und der ersten seitlichen Kamera (215) ein Sichtfeld zwischen 80-120 Grad aufweist.

4. Das medizinische bildgebende Gerät nach Anspruch 1, wobei die erste seitliche Kamera (215) und die zweite seitliche Kamera (245) senkrecht zueinander ausgerichtet sein können und im Wesentlichen 180 Grad oder weniger voneinander entfernt in der zylindrischen Oberfläche der distalen Spitze (1125) an gegenüberliegenden Seiten der zylindrischen Oberfläche von besagter distalen Spitze (115) angeordnet sein können.

5. Das medizinische bildgebende Gerät (205) nach Anspruch 3, wobei einige Bereiche im Sichtfeld der ersten seitlichen Kamera (215) und einige Bereiche im Sichtfeld der vorderen Kamera (130) überlagert sind.

6. Das medizinische bildgebende Gerät (205) nach Anspruch 3, wobei einige Bereiche im Sichtfeld der zweiten seitlichen Kamera (245) und einige Bereiche im Sichtfeld der vorderen Kamera (130) überlagert sind.

7. Das medizinische bildgebende Gerät (205) nach Anspruch 1, wobei das distale Bauteil (115) und das starre langgestreckte Bauteil (155) unter Verwendung eines Mechanismus, ausgewählt aus einer Gruppe umfassend Klebematerial, Löten und Schraubmechanismus, direkt verbunden sind.

8. Das medizinische bildgebende Gerät (205) nach Anspruch 1, wobei das distale Bauteil (115) weiterhin ein vorderes Beleuchtungsmodul (220, 230) zum Beleuchten des von der vorderen Kamera (130) erfassten Bereichs und zwei erste seitliche Beleuchtungsmodule zum Beleuchten des von der ersten seitlichen Kamera (215) erfassten Bereichs umfasst.

9. Das medizinische bildgebende Gerät (205) nach Anspruch 8, wobei ein vorderes Beleuchtungsmodul (220, 230) zwei Lichtquellen auf beiden Seiten der vorderen Kamera (130) umfasst, wobei eine Lichtquelle größer als die andere Lichtquelle ist, falls die vordere Kamera (130) nicht im Zentrum der vorderen Oberfläche angeordnet ist.

10. Das medizinische bildgebende Gerät (205) nach Anspruch 1, wobei die zweite seitliche Kamera (245) relativ zur Längsachse des starren Bauteils (155) geneigt ist.

11. Das medizinische bildgebende Gerät (205) nach Anspruch 1, wobei die vordere Kamera (130) relativ zur Längsachse des starren Bauteils (155) geneigt ist.

12. Das medizinische bildgebende Gerät (205) nach Anspruch 1, wobei die zweite seitliche Kamera (245) und die vordere Kamera (130) relativ zur Längsachse des starren Bauteils (155) geneigt sind.

## Revendications

1. Dispositif d'imagerie médicale (205), comprenant :
un élément allongé rigide (155) ;
un élément distal (115) prévu en une largeur d'approximativement 5,0 à 15 millimètres, ledit élément distal (115) comprend une caméra avant (130) située sur une surface plane avant de l'élément distal (115), une première caméra latérale (215) située sur une première surface latérale de l'élément distal (115) et une seconde caméra latérale (245) située sur une seconde surface latérale de l'élément distal (115, 265), et dans lequel l'élément distal (115, 265) est directement connecté à l'élément allongé rigide (155) de manière rigide ;
**caractérisé en ce que** la première caméra latérale (215) est située à une position plus proche de la surface plane avant que la seconde caméra latérale (245).

2. Dispositif d'imagerie médicale selon la revendication 1, dans lequel chacune de la distance de travail de la caméra avant (130), de la seconde caméra latérale (245) et de la première caméra latérale (215) est dans une plage de 5 à 150 millimètres.

3. Dispositif d'imagerie médicale selon la revendication 1, dans lequel chacune de la seconde caméra latérale (245) et de la première caméra latérale (215) a un champ de vision compris entre 80 et 120 degrés.

4. Dispositif d'imagerie médicale selon la revendication 1, dans lequel la première caméra latérale (215) et la seconde caméra latérale (245) peuvent pointer perpendiculairement l'une à l'autre, et être positionnées essentiellement à 180 degrés ou moins d'écart dans la surface cylindrique de la pointe distale (1125), dans des côtés opposés de la surface cylindrique de ladite pointe distale (115).

5. Dispositif d'imagerie médicale (205) selon la revendication 3, dans lequel certaines zones au niveau du champ de vision de la première caméra latérale (215) et certaines zones au niveau du champ de vision de la caméra avant (130) sont chevauchées.

6. Dispositif d'imagerie médicale (205) selon la revendication 3, dans lequel certaines zones au niveau du champ de vision de la seconde caméra latérale (245) et certaines zones au niveau du champ de vision de la caméra avant (130) sont chevauchées.

7. Dispositif d'imagerie médicale (205) selon la revendication 1, dans lequel l'élément distal (115) et l'élément allongé rigide (155) sont directement connectés en utilisant un mécanisme sélectionné parmi un groupe comprenant du matériau adhésif, une soudure et un mécanisme de vissage.

8. Dispositif d'imagerie médicale (205) selon la revendication 1, dans lequel l'élément distal (115) comprend en outre un module d'éclairage avant (220, 230) pour éclairer la zone capturée par la caméra avant (130) et deux premiers modules d'éclairage latéral pour éclairer la zone capturée par la première caméra latérale (215).

9. Dispositif d'imagerie médicale (205) selon la revendication 8, dans lequel un module d'éclairage avant (220, 230) comprend deux sources lumineuses sur les deux côtés de la caméra avant (130), dans lequel une source lumineuse est plus grande que l'autre source lumineuse au cas où la caméra avant (130) n'est pas positionnée au centre de la surface avant.

10. Dispositif d'imagerie médicale (205) selon la revendication 1, dans lequel la seconde caméra latérale (245) est inclinée par rapport à l'axe longitudinal de l'élément rigide (155).

11. Dispositif d'imagerie médicale (205) selon la revendication 1, dans lequel la caméra avant (130) est inclinée par rapport à l'axe longitudinal de l'élément rigide (155).

12. Dispositif d'imagerie médicale (205) selon la revendication 1, dans lequel la seconde caméra latérale (245) et la caméra avant (130) sont inclinées par rapport à l'axe longitudinal de l'élément rigide (155).
